(19) Europäisches Patentamt
European Patent Office
Office européen des brevets

(11) EP 2 792 355 A1

(12) EUROPEAN PATENT APPLICATION

(43) Date of publication:
22.10.2014 Bulletin 2014/43

(51) Int Cl.:
*A61K 31/40* (2006.01)

(21) Application number: 13164209.2

(22) Date of filing: 17.04.2013

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Albert-Ludwigs-Universität Freiburg
79085 Freiburg (DE)

(72) Inventor: The designation of the inventor has not yet been filed

(74) Representative: Dr. Langfinger & Partner
In der Halde 24
67480 Edenkoben (DE)

(54) **Compounds for use as bromodomain inhibitors**

(57) A compound of formula (1) or a pharmaceutically acceptable salt thereof

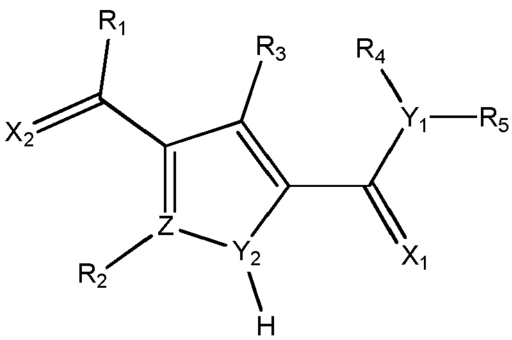

for use in the treatment of diseases or conditions for which a bromodomain inhibitor is indicated.

Fig. 1

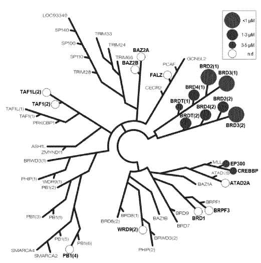

| | BRD2(1) | BRD2(2) | BRD3(1) | BRD3(2) | CREBBP |
|---|---|---|---|---|---|
| $K_d$ (μM) | 0.6 | 2.4 | 0.9 | 1.0 | 3.2 |

| | BRD4(1) | BRD4(2) | BRDT(1) | BRDT(2) | EP300 |
|---|---|---|---|---|---|
| $K_d$ (μM) | 2.7 | 2.3 | 3.4 | 1.4 | 4.1 |

EP 2 792 355 A1

## Description

The present invention relates to compounds for use as bromodomain inhibitors.

Bromodomains are epigenetic reader modules regulating gene transcription by recognizing acetyl-lysine modified histone tails.

The human genome encodes up to 61 different bromodomains (BRDs), present in transcriptional co-regulators and chromatin modifying enzymes including histone acetyl-transferases (HATs) and the Bromodomain extra-terminal domain (BET) family. They are epigenetic mark 'readers' that specifically recognize ε-*N*-acetylated lysine residues ($K_{ac}$).

Drug molecules that target epigenetic mechanisms of gene regulation are attractive as they offer the perspective of modifying processes responsible for dysfunctional malignant states rather than just treating the results thereof.

Bromodomains are small distinct domains within proteins that bind to acetylated lysine residues commonly but not exclusively in the context of histones.

BRDs fold into an evolutionary conserved four anti-parallel helix motif, linked by diverse loop regions of variable length (ZA and BC loops), which define the $K_{ac}$ binding site (Filippakopoulos et al. (2012), Cell 149(1), 214-231). In most BRDs, this site features an asparagine residue mainly responsible for substrate recognition (Owen DJ, et al. (2000) The structural basis for the recognition of acetylated histone H4 by the bromodomain of histone acetyltransferase gcn5p. The EMBO journal 19(22):6141-6149; Umehara T, et al. (2010) Structural basis for acetylated histone H4 recognition by the human BRD2 bromodomain. The Journal of biological chemistry285(10):7610-7618*).* The biological function of BRDs and their potential as therapeutic targets have been thoroughly described in literature (Muller S, Filippakopoulos P, & Knapp S (2011) Bromodomains as therapeutic targets. Expert reviews in molecular medicine 13:e29; Prinjha RK, Witherington J, & Lee K (2012) Place your BETs: the therapeutic potential of bromodomains. Trends in pharmacological sciences 33(3):146-153).

Research on the structural classification of bromodomains has revealed that the BET family of bromodomains has an overall highly targetable recognition site (Vidler LR, Brown N, Knapp S., Hoelder S (2012) Druggability analysis and structural classification of bromodomain acetyl-lysine binding sites. Journal of medicinal chemistry 55(17):7346-7359).

Members of the BET family, namely BRD2, BRD3, BRD4, and BRDT, modulate gene expression by recruiting transcriptional regulators to specific genomic locations. BRD4 and BRD2 have crucial roles in cell cycle control of mammalian cells. Along with BRD3, they are functionally linked to pathways important for cellular viability and cancer signalling and are co-regulators in obesity and inflammation. Specifically, BRD4 has been characterized as a key determinant in acute myeloid leukaemia, multiple myeloma, Burkitt's lymphoma, NUT midline carcinoma, colon cancer, and inflammatory disease. Because of its continued association with $K_{ac}$ in mitotic chromosomes, BRD4 has been postulated to be important for the maintenance of epigenetic memory.

Small molecules that inhibit bromodomains have potential as antiinflammatory, antiviral, and anticancer agents. Anticancer activity is mainly due to down-regulation of the key oncogene *c-MYC.* Recently, cytotoxicity in LAC cells has been related to suppression of the oncogenic transcription factor FOSL1 and its targets.

WO2011/054851 discloses a process for the identification of bromodomain inhibitors.

WO2012/174487 describes bromodomain inhibitors of the general formula

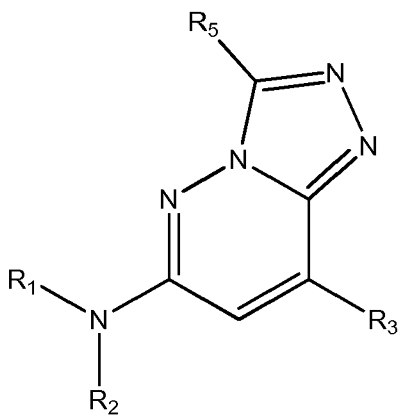

WO2011/054553 describes a benzodiazepine bromodomain inhibitor having the formula

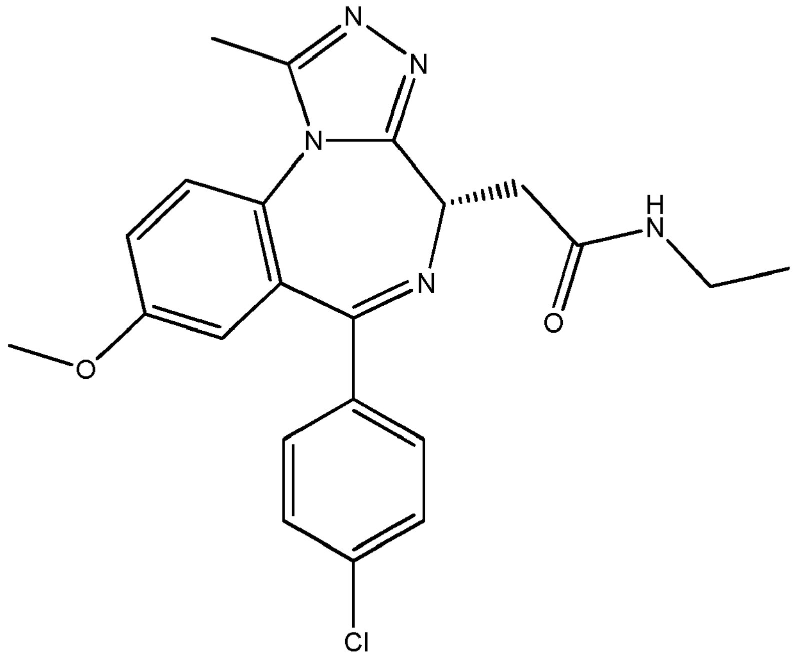

which is also known as I-BET762 and which is in preclinical development as antagonist of the BET bromodomains BRD2, BRD3 and BRD4.

JP 2008/156311 discloses a benzimidazole derivative which is said to be a BRD2 bromodomain binding agent that has utility with respect to virus infection/proliferation.

WO 2009/084693 discloses thienotriazoleodiazepine derivatives having the general formula

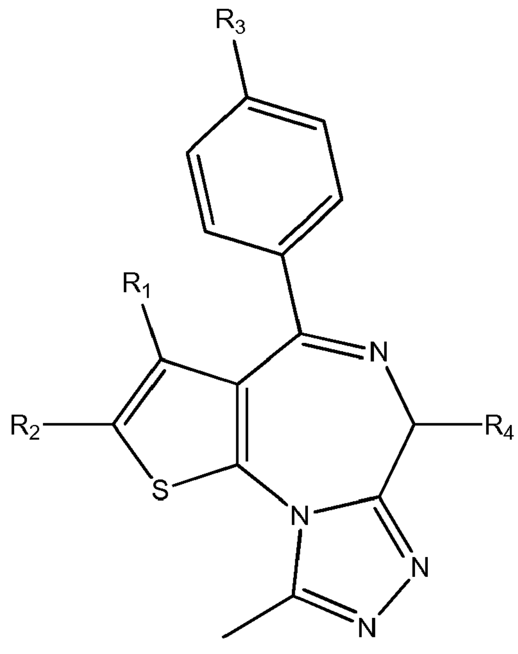

A molecule corresponding to this general formula, namely

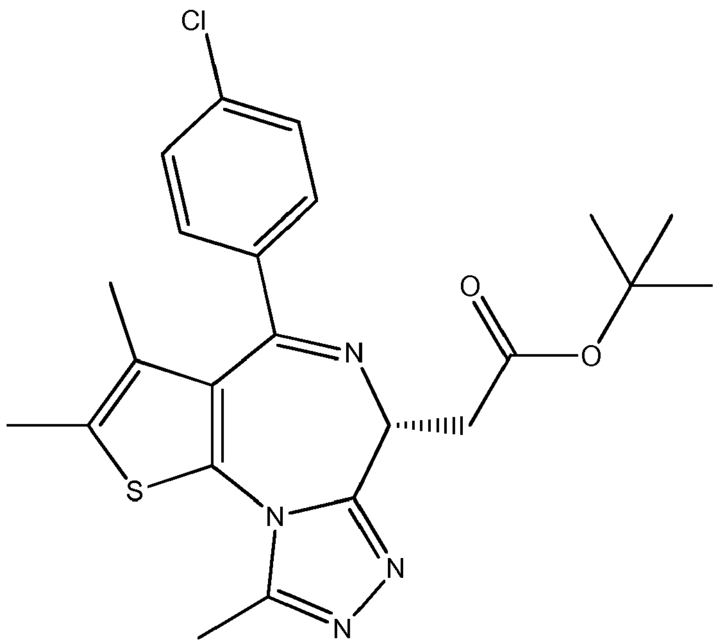

is known as (+)-JQ1 and is in pre-clinical development as bromodomain inhibitor.

Bamborough et al. (2012) J. Med. Chem. 55, 587-596 describe the optimization of phenylisoxazole sulphonamides of the general formula

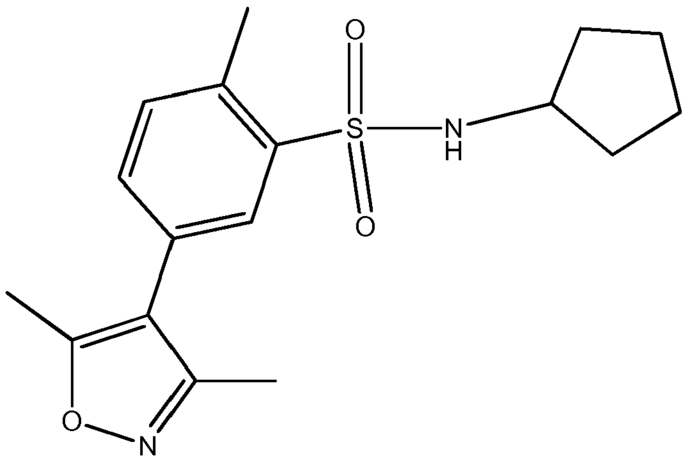

Hewings et al.(2011), J. Med. Chem. 54, 6761-6770 and Dawson et al.(2011), Nature 478 (7370), 529-533 disclose 3,5-dimethylisoxazole derivatives such as a compound known as I-BET151

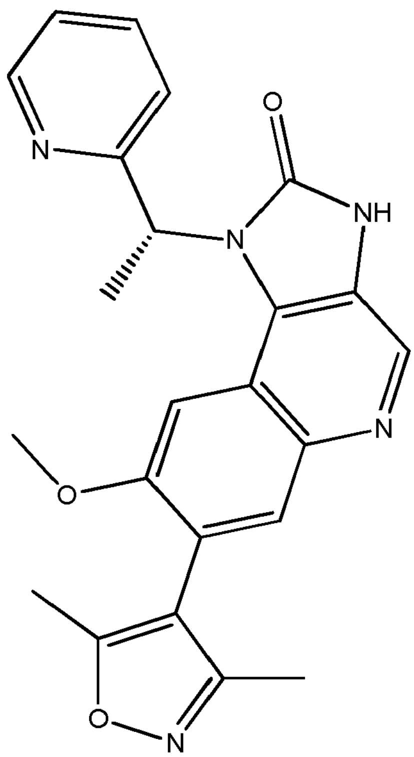

which acts as a $K_{ac}$ mimic to inhibit bromodomains.

Zhao et al., Fragment based drug discovery of 2-thiazolidinones as inhibitors of the histone reader BRD4 bromodomain, J. Med. Chem Just accepted, DOI 10.1021/jm301793a (published online March 26, 2013) disclose 2-thiazolidinones of general formula

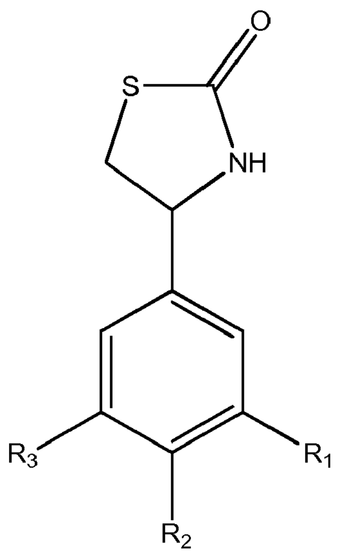

with compounds having a sulphonamide group

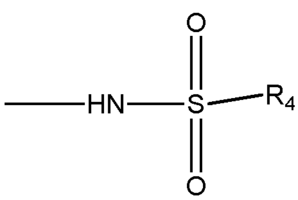

wherein $R_4$ is an aryl or heteroaryl substituent selected from phenyl, thiophene or quinoline, as substituents $R_1$ and/or $R_2$ and/or $R_3$ showing the strongest inhibition of BRD4-BD1.

In this reference it is reported that fragments with very different chemical moieties appear to bind to a similar position in the BRD4-BD1 binding site. Hydrogen bonding to the conserved residue Asn140 of the BRD4 and indirect hydrogen bonding with Tyr97 is believed to be involved. Furthermore, apparently the solvent exposed area around Trp81, an entrance termed as the WPD shelf by Nicodeme et al. (2010), Nature 210, 1119-1123 is also involved in binding.

WO2012/150234 discloses bromodomain inhibitors of formula

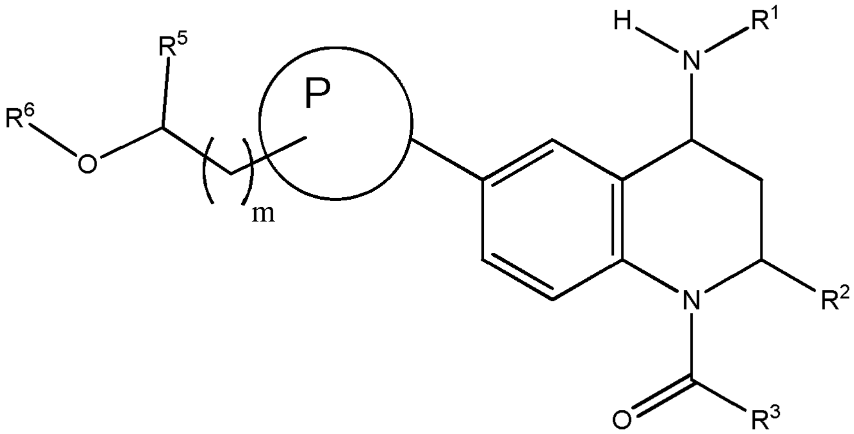

wherein P is a pyrazolyl or triazolyl ring.

WO97/003957 discloses distamycin derivatives of formula

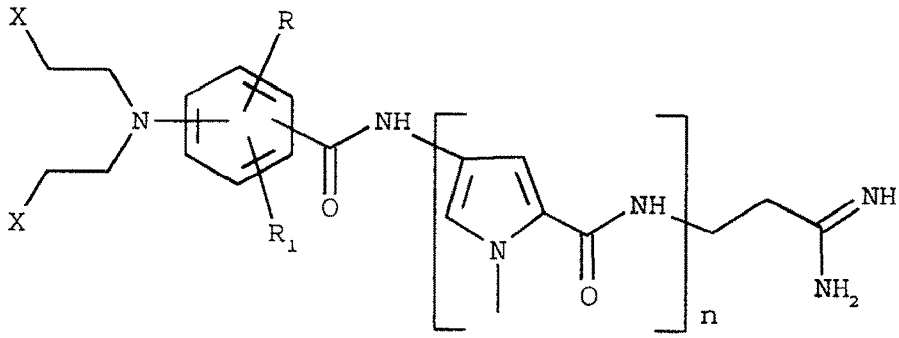

which are active as antitumor or antiviral agents.

As recognition of the acetyllysine of histone is a vital process of epigenetic regulations and as this process is mediated by bromodomains, developing novel bromodomain inhibitors is desirable to provide new approaches for pharmaceutically active ingredients.

Accordingly, it has been an object of the present invention to provide novel bromodomain inhibitors.

This object has been achieved with the compounds as defined in claim 1 for use in the treatment of diseases or conditions for which a bromodomain inhibitor is indicated.

Preferred embodiments of the present invention are set forth in the dependent claims and the detailed description hereinafter.

The present invention relates to compounds of formula (1)

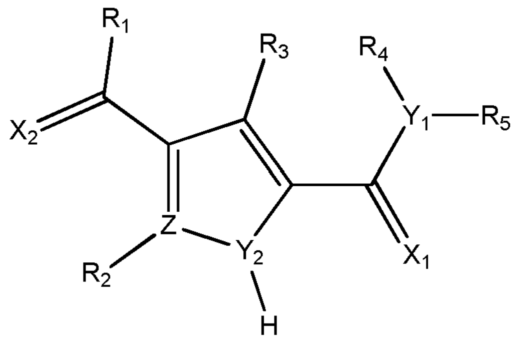

(1)

or a pharmaceutically acceptable salt thereof for use in the treatment of diseases or conditions for which a bromodomain inhibitor is indicated,

wherein

$Y_1$ and $Y_2$, which may be the same or different at each occurrence, are N or P,

$X_1$ and $X_2$, which may be the same or different, are O or S,

Z is C, S, C-$CR_6R_7$- or C-S-

$R_1$ is $C_1$-$C_8$ alkyl, $NHR_8$, $PHR_8$, $NH_2$, $PH_2$, a substituted or unsubstituted $C_1$-$C_8$-hydroxyalkyl group, or a substituted or unsubstituted $C_1$-$C_6$-alkoxy group,

$R_2$ is hydrogen, a halogen atom, a cyano group or a thiocyanate or isothiocyanate group or a $C_1$-$C_8$ alkyl group,

$R_3$ is hydrogen, $C_1$-$C_8$ alkyl. a substituted or unsubstituted $C_3$-$C_8$-carbocyclyl group, a substituted or unsubstituted $C_3$-$C_8$-heterocarbocyclyl group comprising up to two heteroatoms selected from N, O and S, , or a 5- or 6-membered aryl or heteroaryl group,

$R_4$ and $R_5$, which may be the same or different at each occurrence, are hydrogen, a $C_1$-$C_8$ alkyl group, a $C_1$-$C_8$ alkoxy group, a $C_1$-$C_8$ hydroxyalkyl group, a substituted or unsubstituted $C_3$-$C_{14}$ cycloalkyl or heterocycloalkyl group or an unsubstituted or substituted $C_3$-$C_{14}$ aryl or heteroaryl group, which cycloalkyl, heterocycloalkyl, aryl or heteroaryl group may be bound to $Y_1$ directly or through a spacer of a $C_1$-$C_8$ alkylene group.

$R_6$ and $R_7$, which may be the same or different, are hydrogen or a $C_1$ to $C_8$ alkyl group and

$R_8$ is hydrogen or a $C_1$ to $C_8$ alkyl group.

The term $C_1$-$C_8$ alkyl group, when used herein, represents a linear or branched hydrocarbon group with 1 to 8 carbon atoms. Examples are methyl, ethyl, *i*-propyl, n-propyl, *i*-butyl, n-butyl and *t*-butyl, to name only a few representatives.

The term $C_1$-$C_8$ hydroxyalkyl group, when used herein, represents a linear or branched $C_1$-$C_8$-alkyl group in which one or more of the hydrogen atoms are replaced by a hydroxy group.

The term $C_1$ to $C_8$ alkoxy group, when used herein, represents a derivative of an alkyl group comprising at least one O-C bond. Representative examples are $OCH_3$, $OC_2H_5$, $OC_3H_7$ and $OC_4H_9$.

The term 5- or 6-membered substituted or unsubstituted carbocyclyl, heterocarbocyclyl, aryl, or heteroaryl group represents a five or six membered ring which may comprise carbon atoms and, optionally, one or more heteroatoms, preferably selected from O, S, N and P, more preferably from O, S and N, and particularly preferred from O and N. The ring may be saturated or it may carry double bonds, in particular it may be aromatic.

Preferred carbocyclyl rings are cyclopentyl and cyclohexyl, preferred heterocarbocyclyl groups are cyclopentyl and cyclohexyl groups in which one or more of the ring carbon atoms are replaced by a heteroatom selected from O, S, N and P as defined above.

Phenyl groups are representatives of six membered aromatic rings and 5-or six membered heteroaryl groups are preferably selected from the following ring systems.

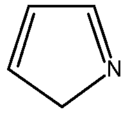

2*H*-pyrrole

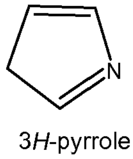

3*H*-pyrrole

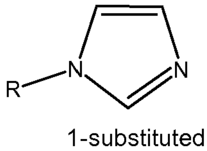

1-substituted 1*H*-imidazole

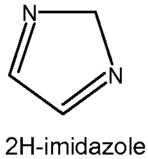

2H-imidazole

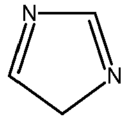

4*H*-imidazole

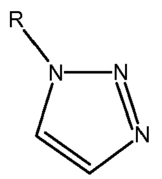

1-substituted-1*H*-1,2,3-triazole

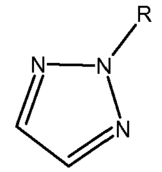

2-substituted-2H-1,2,3-triazole

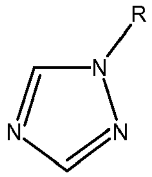

1-substituted-1*H*-1,2,4-triazole

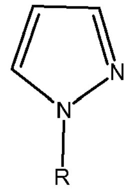

1-substituted-1*H*-pyrazole

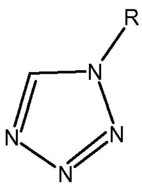

1-substituted 1H-1,2,3,4-tetrazole

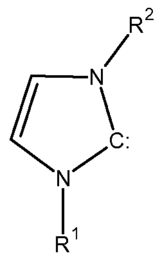

1,3-disubstituted imidazol-2-ylidene

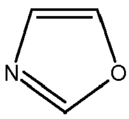

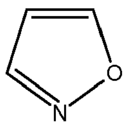

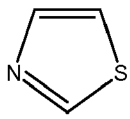

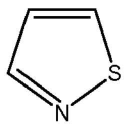

oxazole isoxazole thiazole isothiazole

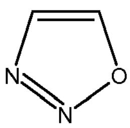

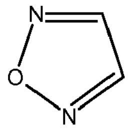

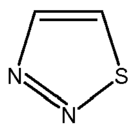

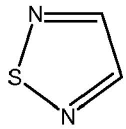

1,2,3-oxadiazole 1,2,5-oxadiazole 1,2,3-thiadiazole 1,2,5-thiadiazole

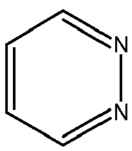

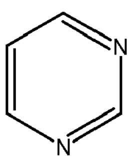

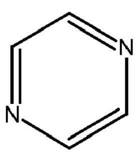

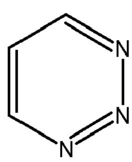

pyridazine pyrimidine pyrazine 1,2,3-triazine

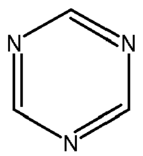
1,3,5-triazine

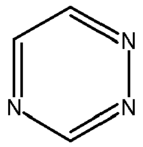
1,2,4-triazine

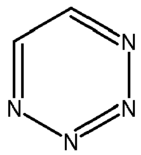
1,2,3,4-tetrazine

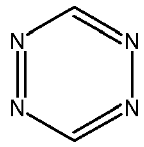
1,2,4,5-tetrazine

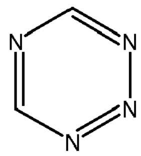
1,2,3,5-tetrazine

All rings may be substituted or unsubstituted, i.e. one or more of the hydrogen atoms attached to the ring atoms in the unsubstituted representatives may be replaced by substituents consisting of halogen, alkyl, alkoxy, amino, cyano, alkenyl, alkynyl, arylalkyl, aryl, and heteroaryl groups.

The term substituted or unsubstituted $C_3$-$C_{14}$ cycloalkyl or heterocycloalkyl group or an unsubstituted or substituted $C_3$-$C_{14}$ aryl or heteroaryl group, when used herein, represents a ring system, which may be a fused ring system, comprising the indicated number of carbon atoms, and in which in the heterohydrocarbyl and heteroaryl groups one or more of the ring carbon atoms is replaced by a heteroatom selected from O, N, S and P, preferably O, N and S and most preferably N or O. All rings may be substituted or unsubstituted, i.e. one or more of the hydrogen atoms attached to the ring atoms in the unsubstituted representatives may be replaced by substituents consisting of halogen, alkyl, alkoxy, amino, cyano, alkenyl, alkynyl, arylalkyl, aryl and heteroaryl groups. Representative examples are cyclopropyl, cyclopentyl, cyclopentenyl, cyclopentadienyl, cyclohexyl, cycloheptyl, cyclooctyl, phenyl, napthyhyl and anthracenyl, which may all comprise one or more heteroatoms and one or more substituent groups as defined above.

A first preferred group of compounds for use in accordance with the present invention are compounds of formula (1) wherein $Y_1$ or $Y_2$, more preferably $Y_1$ and $Y_2$ are N.

Furthermore, preferably at least one of $X_1$ or $X_2$, preferably both $X_1$ and $X_2$ represent an oxygen atom.

In accordance with another preferred embodiment of the present invention Z is C or C-S-, preferably C. The respective compounds, in case $Y_2$ is N, are derivatives of pyrrole and isothiazole (i.e. in the latter case the sulphur atom in the ring is attached to the nitrogen atom).

$R_2$ is hydrogen, a halogen atom, a cyano group or a thiocyanate or isothiocyanate group or a $C_1$ to $C_8$ alkyl group. In certain cases $C_1$ to $C_6$ alkyl groups, halogen atoms or cyano groups have shown to be advantageous. Halogen atoms are selected from fluorine, chlorine, bromine and iodine atoms, with fluorine, chlorine and bromine being preferred. Methyl or ethyl are preferred representatives of alkyl groups, particularly preferred is a methyl group.

In accordance with another preferred embodiment $R_3$ is a $C_1$-$C_8$ alkyl group, preferably a $C_1$ -$C_6$ alkyl group and particularly preferred $R_3$ is a methyl, ethyl, propyl, or isopropyl group. Other preferred examples for $R_3$ are $C_3$-$C_8$-cycloalkyl or $C_3$-$C_8$ heterocycloalkyl groups with up to two heteroatoms selected from N, O or S or 5-or 6-membered aryl rings, preferably phenyl, or 5- or 6-membered heteroaryl rings as defined above, with pyrrole, furan, imidazole, thiophene or thiophene as preferred examples. All ring systems may be substituted or unsubstituted with halogen, cyano, thiocyanate, isocyanate or $C_1$-$C_8$ alkyl groups, with halogen, in particular fluorine, chlorine or bromine and preferably chlorine being preferred. Halogen or cyano substituted phenyl or pyridine may be mentioned as preferred here.

In accordance with still another preferred embodiment of the present invention $R_4$ and/or $R_5$ are hydrogen or a substituted or unsubstituted $C_3$-$C_{14}$ aryl group, more preferably a substituted phenyl group.

In accordance with another embodiment, one of $R_4$ and $R_5$ is a hydrogen atom and the other substituent is a $C_1$-$C_8$ alkyl group (preferably methyl or ethyl) or a substituted or unsubstituted $C_3$-$C_{14}$ aryl group, more preferably a substituted phenyl group.

For some indications compounds in which $R_4$ and/or $R_5$ is a phenyl group bearing a sulphonamido substituent (which may be in o-, m- or p-position relative to the carbon atom bound to $Y_1$ with a substituent in m-position showing particularly good results in some cases) have shown to yield particularly good results and a particularly preferred example for $R_4$ and/or $R_5$ is represented by formula 2

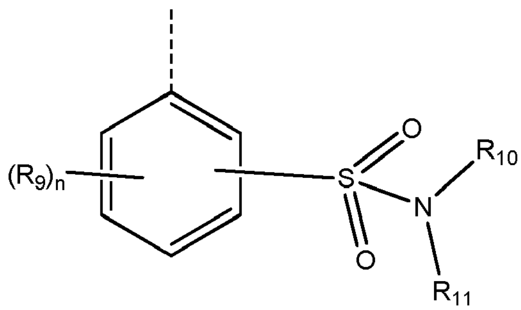
(2)

wherein $R_9$ is halogen, OH, SH, $SR_6$, cyano, thiocyanato, isothiocyanato, $NR_6R_7$, a $C_1$-$C_8$ alkyl group, a $C_2$-$C_8$ alkylene group, a $C_2$-$C_8$ alkynyl group or a $C_1$ to $C_8$ alkoxy group, wherein $R_6$ and $R_7$ are as previously defined and n is an integer of from 0 to 4, preferably of from 1 to 3.

$R_{10}$ and $R_{11}$, which may be the same or different, are hydrogen, a $C_1$-$C_8$ alkyl group or a substituted or unsubstituted $C_3$-$C_{14}$ cycloalkyl group, heterocycloalkyl, aryl or heteroaryl group or wherein $R_{10}$ and $R_{11}$ may form together a cyclocarbyl, heterocyclocarbyl, aryl or heteroaryl ring, each with 3 to 14 carbon atoms.

Particularly preferred are compounds with $R_9$ being a hydroxy or methoxy group and $R_{10}$ and/or $R_{11}$ being a $C_1$-$C_4$ alkyl group, in particular a methyl or ethyl group or wherein $R_{10}$ and $R_{11}$ together form a ring system, preferably a $C_3$-$C_9$ cyclocarbyl group, especially preferably cyclopentyl, cyclohexyl or cycloheptyl.

In accordance with a further preferred embodiment one of $R_4$ and $R_5$ is hydrogen.

A first group of particularly preferred groups for use in accordance with the present invention are compounds of formula (3)

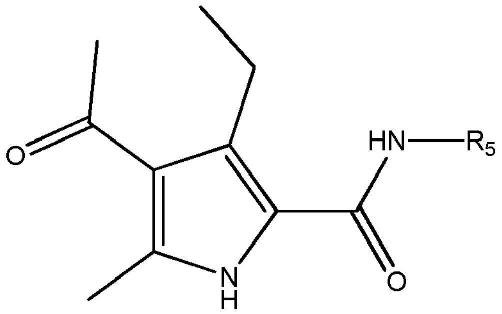

(3)

wherein $R_5$ has the meaning as described above.

A particularly preferred compound for use in accordance with the present invention is represented by formula (4)

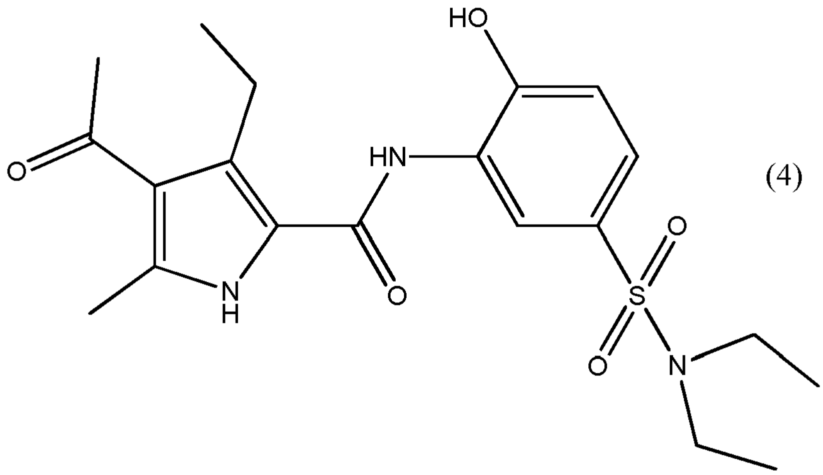

(4)

which has shown to be particularly effective for the intended use.

In accordance with the present invention the compounds of formula (1) may be used in the form of their pharmaceutically acceptable salts. The term. The skilled person is aware of pharmaceutically acceptable salts of compounds of formula (1) and will select the appropriate salt based on his professional experience and knowledge. Suitable pharmaceutically acceptable salts can include acid or base addition salts. For a review on suitable salts see Berge et al. (1977) J. Pharm.Sci.66:1-19. Typically, pharmaceutical acceptable salts may be readily prepared by using a desired acid or base as appropriate. The resultant salt may precipitate from solution and be collected by filtration or may be recovered by evaporation of the solvent.

A pharmaceutically acceptable base addition salt can be formed by reaction of a compound of formula (1) with a suitable inorganic or organic base, optionally in a suitable solvent, to give the base addition salt which is usually isolated, for example by crystallization and filtration. Pharmaceutically acceptable base salts include ammonium salts, alkali metal salts, alkaline earth metal salts and salts with organic basis, in particular salt with primary, secondary and tertiary amines, e.g. isopropyl amine, diethylamine, ethanolamine, trimethylamine and dicyclohexylamine.

The pharmaceutically acceptable acid addition salt can be formed by reaction of a compound of formula (one) with a suitable inorganic or organic acid, opportunity in a suitable solvent such as an organic solvent, to give the solvent which is usually isolated, for example, by crystallization and filtration. Pharmaceutically acceptable acid addition salt of a compound of formula (1) can comprise or be, for example, a nitrate, sulphate, hydrobromide, hydrochloride, phosphate, maleate, acetate, propionate, fumarate, citrate, tartrate, salicylate, aspartate, succinate, benzoate, p-toluenesulphonate, methanesulphonate, naphthalenesulphonate, ethanesulphonate or hexanoate salt.

In accordance with the present invention the pharmaceutically acceptable salts includes all possible stoichiometric and non-stoichiometric forms of the salts of the compounds of formula (1).

The compounds of formula (1) form complexes with solvents in which they are reacted or from which they are precipitated or crystallized, which complexes are generally referred to as solvates. In accordance with the present invention on possible stoichiometric and non-stoichiometric forms of the solvates of the compounds of formula (1) are included.

The present invention also encompasses all prodrugs of the compounds of formula (1) and pharmaceutically acceptable salts thereof, which upon administration to the recipient are capable of providing (directly or indirectly) a compound of formula (1) or a pharmaceutically acceptable salt thereof or an active metabolite or aggressive you thereof. In this regard, reference is made to Burger's Medicinal Chemistry and drug discovery, 5th Ed., Vol. 1, Principles and Practice, to which reference is made here for further details.

The compounds of formula (1) may be in crystalline or amorphous form. Furthermore, some of the crystalline forms may exist in different morphological conformations, which are included within the scope of the present invention. Different morphological forms (polymorphic forms) of the compounds of formula (1) can be characterized and differentiated analytical techniques known to the person skilled in the art so that no further details are necessary here.

The compounds of formula (1) may also exist in one of several tautomeric forms and in accordance with the present invention individual tautomers or mixtures thereof are encompassed.

The compounds of formula (1) or their pharmaceutically acceptable salts are provided for use in the treatment of diseases or conditions for which a bromodomain inhibitor is indicated.

Bromodomain inhibitors are believed to be useful in the treatment of a variety of diseases or conditions related to systemic or tissue inflammation, inflammatory responses to infection or hypoxia, cellular activation and proliferation, lipid metabolism, fibrosis and in the prevention and treatment of viral infections.

Bromodomain inhibitors may thus be useful in the treatment of a bite variety of chronic autoimmune and inflammatory conditions such as rheumatoid arthritis, osteoarthritis, acute gout, psoriasis, systemic lupus erythematosus, multiple sclerosis, inflammatory bowel disease, asthma, chronic obstructive airways disease, pneumonitis, myocarditis, pericarditis, myositys, eczema, dermatitis, alopecia, vitiligo, bullous skin diseases, nephritis, vasculitis, atherosclerosis, Alzheimer's disease, depression, retinitis, uveitis, scleitis, hepatitis, pancreatitis, primary biliary cirrhosis, sclerosing, cholangitis, Addison's disease, hypophysitis, thyroiditis, type I diabetes and acute rejection of transplanted organs.

The compounds in accordance with formula 1 for use in diseases or conditions for which a bromodomain inhibitor is indicated may also be useful in the prevention or treatment of diseases or conditions which involve inflammatory responses to infections with bacteria, viruses, fungi, parasites or their scenes, such as sepsis, sepsis syndrome, septic shock, endotoxaemia, systemic inflammatory response syndrome (SIRS), multi-organ dysfunction syndrome, toxic shock syndrome, acute lung injury, adult respiratory distress syndrome, acute renal failure, fulminant hepatitis, burns, acute pancreatitis, post-surgical syndromes, sarcoidosis, Herxheimr reactions, encephalitis, myelitis, meningitis, malaria, and SIRS associated with viral infections such as influenza, herpes zoster, herpes simplex, and coronavirus.

The compounds of formula (1) may also be used in the treatment of disorders of the lipid metabolism while the regulation of APO-A1 such ashypercholesterolemia, atherosclerosis, and Alzheimer's disease.

Furthermore, the compounds of formula (1) may be used in the prevention or treatment of conditions associated with ischemia-reperfusion injury such as myocardial infarction, cerebrovascular ischemia (stroke), acute coronary syndromes, renal reperfusion injury, organ transplantation, coronary artery bypass grafting, cardio-pulmonary bypass procedures and pulmonary, renal, hepatic, gastro-intestinal or peripheral limb embolism.

Bromodomain inhibitors may further be useful in the treatment of fibrotic conditions such as idiopathic pulmonary fibrosis, renal fibrosis, postoperative stricture, keloid formation, scleroderma and cardiac fibrosis.

A further indication for bromodomain inhibitors is the prevention and treatment of viral infections such as herpes virus, human papilloma virus, adenovirus, poxvirus and other DNA viruses.

The compounds of formula (1) are useful in the treatment of cancer, including but not limited to types of cancer included in the NC160 panel, such as bladder cancer, brain cancer, breast cancer, cervical carcinoma, colorectal cancer, oesophageal cancer, gastric cancer, head and neck cancer, leukaemia, lymphoma, NSCLC cancer (non-small cell lung carcinoma), ovarian cancer, pancreatic cancer, sarcoma, SCLC cancer (small cell lung cancer), melanoma, renal cancer, prostate cancer and hepatocellular carcinoma. including haematological (such as leukaemia), epithelial including lung, breast and colon carcinomas, midline carcinomas, mesenchyme, hepatic, renal and neurological tumours.

Preferred uses of the compounds of formula (1) are the treatment of chronic autoimmune or inflammatory

conditions, cancer or viral diseases.

The compounds of formula (1) can also be preferably used for the induction of apoptosis in leukaemia cells or for the treatment of a disease or condition which is obesity or a kidney malfunction, in particular the compounds of formulae (3) and (4) have shown good efficiency in the induction of apoptosis in leukaemia cells.

Furthermore, the compounds of formula (1) could be also effective as male contraceptives.

Compounds of formula (1) can also be used in the treatment of diseases or conditions related to bromodomains CREBBP and EP300. By way of example, acute myeloid leukaemia, acute lymphoblastic leukaemia, non-Hodgkin lymphoma, prostate cancer and spinal and bulbar muscular atrophy may be mentioned in this regard.

In the course of the studies leading to the present invention it has been recognized that the compounds of formula (1) in particular are effective in inhibiting the bromodomains of the BET family, including BRD2-BD1, BRD2-BD2, BRD3-BD1, BRD3-BD2, BRD4-BD1, BRD4-BD2, BRDT-BD1 and BRDT-BD2 as well as the bromodomains CREBBP and EP300, with a particularly good effect in the inhibition of the mentioned bromodomains of the BET family. In some cases, particularly good results were obtained in the inhibition of bromodomains BRD2-BD1, BDRD3-BD1 and BRD3-BD2 whereas in other cases efficient inhibition of BRD4-BD1 was observed.

A similar behaviour was observed for the compounds of formula (3), in particular where $R_5$ is an alkyl group. These compounds share a core with the compound of formula (4) but lack the sulphonamide substituent.

In accordance with the present invention, the compounds of formula (1) as well as their pharmaceutically acceptable salts may be administered as such or may be presented as active ingredients in a pharmaceutical composition.

Accordingly, a further embodiment of the present invention relates to a pharmaceutical composition comprising at least one compound of formula (1) or a pharmaceutically acceptable salt thereof for use in the treatment of diseases or conditions for which a bromodomain inhibitor is indicated and one or more pharmaceutically acceptable carriers, diluents or excipients.

The carriers, diluents or excipients used in pharmaceutical compositions must be acceptable in the sense of being compatible with the other ingredients of the composition and not detrimental to the recipient.

Such pharmaceutical compositions may be obtained by mixing a compound of formula (1) or a pharmaceutically acceptable salt thereof, with one or more pharmaceutically acceptable carriers, diluents or excipients.

Since the compounds and pharmaceutically acceptable salts thereof are intended for use in pharmaceutical compositions it is apparent that they are preferably provided in substantially fewer form, i.e. with a degree of impurities as low as possible. Preferably the purity is at least 60%, more preferably at least 75% and most preferably at least 98% (in each case per cent by weight).

Pharmaceutical compositions may be presented in unit dose forms containing a predetermined amount of active ingredient per unit dose. Preferred unit dosage compositions are those containing a daily dose or sub-dose, or an appropriate fraction thereof, of an active ingredient. Such unit doses may therefore be administered more than once a day. Preferred unit dosage compositions are those containing a daily dose or sub-dose as recited about.

The pharmaceutical compositions may be adapted for administration by any appropriate route, for example by the oral, rectal, inhaled, intranasal, topical, vaginal or parenteral route.

The skilled person will select the appropriate administration route based on the conditions of the specific case. The manufacture of the appropriate administration forms is known to the skilled person so that no further details are necessary here.

When desired or necessary, suitable binders, glidants, lubricants, sweetening agents, flavours, disintegrating agents and colouring agents can be included in the pharmaceutical composition. The skilled person will select the appropriate additives based on his professional experience and in adaptation to the specific application.

The pharmaceutical compositions in accordance with the present invention may comprise more than one active ingredient, e.g. more than one compound of formula (1) or a pharmaceutically acceptable salt thereof or a combination of a compound of formula (1) with other active ingredients or their pharmaceutically acceptable salts.

In particular combinations of bromodomain inhibitors of formula (1) and histone deacetylase inhibitors (HDAC inhibitors) provide a very interesting property spectrum and combination of efficiencies.

Various HDAC inhibitors useful to be combined with the compounds of formula (1) have been investigated and described in the literature and are known to the skilled person. Vorinostat and Romidepsin have been approved by the US FDA for cutaneous T-cell lymphoma.

Panobinostat, valproic acid and belinostat are in phase III clinical trials for various types of cancer.

Mocetinostat, Abexinostat, Entinostat, Resminostat, Givinostat, Qusinostat and SB 939 are in phase II clinical trials.

CUDC-101, AR-42, ACY-1215, Kevetrin, Trichostatin A are in early stage development as HDAC inhibitors.

All the aforementioned HDAC inhibitors can principally be combined with the compounds of formula (1) in accordance with the present invention to obtain pharmaceutical compositions.

The compounds of formula (1) for use in accordance with the present invention the present a novel clause of interesting active molecules in the epigenetic yield and in particular may be considered as a representative of a new

class of bromodomain inhibitors of the BET family featuring a 4-acyl pyrrole moiety.

Certain compounds of formula (1) are commercially available, i.e. from Enamine Ltd.

The skilled person is aware of suitable methods for the synthesis of compounds of formula (1) so that there is no further description necessary here.

The compounds of formula 1 or a pharmaceutically acceptable salt thereof may be used in the manufacture of a medicament for the treatment of diseases or conditions which a bromodomain inhibitor is indicated. In other embodiments, there is provided the use of a compound of formula (one) or a pharmaceutically acceptable salt thereof in the manufacture of a medicament for the treatment of a chronic autoimmune and/or inflammatory condition or in the treatment of cancer.

In a further embodiment there is provided a method for treatment of a disease or condition, for which a bromodomain inhibitor is indicated, in a subject in need thereof which comprises administering a therapeutically effective amount of a compound of formula (1) or a pharmaceutically acceptable salt thereof.

In one embodiment the subject in need of treatment is a mammal.

The term effective amount means an amount of a drug or pharmaceutical agent that will elicit the biological or medical response of a tissue, system, animal or human that is being sought, for instance by a researcher or a clinician. Furthermore, the term therapeutically effective amount means any amount which, as compared to a corresponding subject whereas not received such a mound, results in improved treatment, healing, prevention or amelioration of a disease, disorder, or side effect, or a decrease in the rate of advancement of a disease or disorder. The term also includes within its scope amounts effective to enhance normal physiological function.

The following examples represent embodiments of the present invention without limiting the scope thereof. The skilled person will easily recognize that he can modify the structure of the compounds in the working examples in an appropriate manner without deviating from the core of the present invention.

**Example** 1

The compounds of formula (4) and the compound of formula (3) with $R_5$ being methyl which were studied in the working examples were obtained from Enamine Ltd (compounds of formulae (3) and (4)) and InterChim Ltd. (compound of formula (4)).

**Protein preparation, crystallization and structure determination**

BRD4-BD1 was expressed and purified as described previously

(Filippakopoulos et al, (2012) Cell 149(1): 241-231) with the exception of the final buffer for crystallization and ITC analysis (20 mM Hepes/NaOH pH 7.5, 150 mM NaCl). Crystals were grown in the presence of 3.5 M Na-Formate pH 7.5 at a protein concentration of 10 mg/ml and a ligand concentration of 2 mM added directly to the protein prior to crystallization from a 100 mM stock solution in DMSO. Data were collected at 100K using either a Rigaku HF-007 rotating anode X-ray generator equipped with VariMaxHF optics and a Saturn944 CCD detector or a mar345 image plate respectively at Å = 1.54179 Å or at the PXI beamline at the Swiss Light Source at $\lambda$ = 1.000 Å with a Pilatus detector. Data processing and reduction was done with iMOSFLM (Leslie AGW PH (2007), Evolving Methods for Macromolecular Crystallography 245, 41.51), POINTLESS, and SCALA ( Kabsch W (2010) Xds. Acta crystallographica. Section D, Biological crystallography 66(Pt 2):125-132; Bruker (2008) SADABS, SAINT and XPREP (Bruker AXS Inc., Madison, Wisconsin, USA); Collaborative Computational Project N (1994) The CCP4 suite: programs for protein crystallography. Acta crystallographica. Section D, Biological crystallography 50(Pt 5):760-763), or with XDS, XSCALE, XDSCONV (Evans P (2006) Scaling and assessment of data quality. Acta crystallographica. Section D, Biological crystallography 62(Pt 1):72-82), and XPREP(Evans PR (2011) An introduction to data reduction: space-group determination, scaling and intensity statistics. Acta crystallographica. Section D, Biological crystallography 67(Pt 4):282-292). BRD4•ligand complexes crystallized in space group $P2_12_12_1$. The structures were solved by molecular replacement with PHASER (McCoy AJ, et al. (2007) Phaser crystallographic software. Journal of applied crystallography 40(Pt 4):658-674) with apoBRD4 as search model (internal data) yielding one molecule per asymmetric unit. Compounds were modelled into 2Fo-Fc electron density maps using AFITT-CL (version 2.1.0, OpenEye Scientific Software, Inc., Santa Fe, NM, USA.) Model building and real space refinement was done with COOT (Murshudov GN, Vagin AA, & Dodson EJ (1997) Refinement of macromolecular structures by the maximum-likelihood method. Acta crystallographica. Section D, Biological crystallography 53(Pt 3):240-255) reciprocal space refinement against the calculated data was done with Refmac5, as implemented in the CCP4 suite (Murshudov GN, et al. (2011) REFMAC5 for the refinement of macromolecular crystal structures. Acta crystallographica. Section D, Biological crystallography 67(Pt 4):355-367; Vaguine AA, Richelle J, & Wodak SJ (1999) SFCHECK: a unified set of procedures for evaluating the quality of macromolecular structure-factor data and their agreement with the atomic model. Acta crystallographica. Section D, Biological crystallography 55(Pt 1): 191-205). Final structure validation was done with procheck/sfcheck (Wlodek S, Skillman AG, & Nicholls A (2006) Automated ligand placement and refinement with a combined force field and shape potential. Acta crystallographica. Section D, Biological crystallography 62(Pt 7):741-749).

Isothermal titration calorimetry

ITC experiments for the determination of the dissociation constant $K_d$ were done with a Microcal VP-ITC microcalorimeter (GE Healthcare) at 25 °C using ligand concentrations between 10 and 50 μM in the sample cell and BRD4 concentrations between 120 and 600 μM in the injection syringe. Data were obtained in discrete titration experiments with an injection volume of 12 μl per injection. Subsequently, the heats per injection were calculated as integrals and after normalization against the molar concentrations plotted against the molar ratio as implemented in Microcal Origin. Finally, data were fitted according to a single set of sites binding model with

$$(1) \quad Q = nM_t \times \Delta HV_0 \times \frac{1}{2} \times \left(1 + \frac{X_t}{nM_t} + \frac{1}{nKM_t} - \sqrt{\left(1 + \frac{X_t}{nM_t} + \frac{1}{nKM_t}\right)^2 - \frac{4X_t}{nM_t}}\right)$$

as function to calculate the overall sum of heat of the titration and with

$$(2) \quad \Delta Q(i) = Q(i) + \frac{dV_i}{V_0} \times \left(\frac{Q(i)+Q(i-1)}{2}\right) - Q(i-1)$$

(According to Microcal's manual "ITC Data Analysis in Origin" (September 1998)) as function describing the sum of heat of each individual injection. A correction term was included to compensate for displacement of volume of the sample cell in the course of subsequent injections according to the manufacturer's manual "ITC Data Analysis in Origin" (Microcal).

The dissociation constant $K_d$ is commonly used to describe the affinity between a ligand L and a protein, i.e. how tightly a ligand binds to a particular protein. The dissociation constant has molar units which correspond to the concentration of ligand at which the binding site on a particular protein is half occupied, i.e. the concentration of the ligand at which the concentration of protein with ligand bound equals the concentration of protein with no ligand bound. The smaller the dissociation constant, the higher the affinity between ligand and protein.

For the compound of formula (4) the dissociation constant $K_d$ for BRD4-BD1 was determined to be 0.3 μM whereas the respective value for the compound of formula (3) with $R_5$ being methyl was 20 μM. The compound of formula (4) thus showed an improved affinity compared to the compound of formula (3) with $R_5$ being methyl.

The concentration required to reduce the population of a culture of HL-60 cells by 50% ($GI_{50}$) was 14 μM for the compound of formula (4).

**Bromodomain profiling**

Bromodomain profiling was carried out on the basis of BROMOscan™. This platform accounted for the indirect determination of the dissociation constants between 19 bromodomains and the compound of formula (4), by binding competition against a reference immobilized ligand.

The overall structure of the complex of the compound of formula (4) and BRD4-BD1 revealed the already well-characterized bromodomain fold with a bundle of four α-helices, interconnected by three loops of different length. The termini of the helices bundle are flanked by elongational loops, which tightly pack against the protein core producing a compact and rather rigid structure.

The compound of formula (4) is bound in a pocket located at the end of the longitudinal axis running through the helix bundle which points towards the N-terminus. Consequently, it occupies the same pocket as the native $K_{ac}$ substrate.

Moreover, it mimics the $K_{ac}$ interaction with BRD4-BD1 by positioning the 4-acyl substitution in the pyrrole ring toward the highly conserved Asn140, thus engaging in hydrogen bond interactions with Asn140 and the equally conserved water molecule that bridges to the conserved Tyr97. The pyrrole ring is located deep in the recognition pocket, and complements the hydrophobic pocket defined by the four conserved waters with a 5-methyl substitution. The surface complementarity between the ligand and the recognition pocket is further achieved by the 3-ethyl substitution in the pyrrole ring. The presence of the heteroatom in the core of the compound of formula (4) allows for a key hydrogen-bond donor interaction with the proline's backbone in position 82. Such interaction has not been described previously, and may have an important role in fixating the compound in the recognition site.

Apart from this major determinant of ligand recognition by BRD4-BD1, the compound of formula (4) also explores another patch of interactions.

The phenylsulphonamide moiety is placed along the ZA channel, such that a T-shaped CH-π interaction with Trp81 is established, whereby a perfect orthogonal orientation of both aromatic systems is created withTrp81 directly pointing towards the centre of the phenyl moiety of the compound of formula (4). Such interactions have already been reported in other drug-protein interactions. In addition, Leu92 serves as lid from the opposing side and together with Trp81 it forms a special configuration which will be referred to hereinafter as WL trap.

The compound of formula (3) with $R_5$ being a methyl group represents a shortened fragment of the compound of formula (4) lacking the phenylsulphonamide extension, and consequently, it perfectly fits into the site around Asn140

and the conserved waters; yet it fails to establish the interaction with the WL trap.

It appears that the WL trap adds to the specificity of the binding. Notably, both Trp81 and Leu92 are conserved within the BET bromodomain family.

The target selectivity of the compound of formula (4) within the human BRD family was assessed by means of BROMOscan™. The binding of the compound to at least one representative protein of each BRD family was assessed. The results are summarized in Figure 1. Figure 1 shows the selectivity profile of the compound of formula (4) within the human BRD family. Sphere size and colour indicate the binding affinity of the compound of formula (4) to the specific BRDs, as observed in the BROMO*scan*™ assay. Additionally, binding to PB1-BD4 was assessed by local ITC measurements. The dissociation constant ($K_d$) corresponding to each BRD in complex with the compound of formula (4) is indicated. The sequence similarity-based phylogenetic tree of the BRD family was extracted from the profiling visualization tool TREE*spot*™ and reproduced under permission of DiscoveRx Corp. The reproducible value of the dissociation constant ($K_d$) of the compound of formula (4) in complex with BRD4-BD1 was shown to be 0.16 μM (n = 2) in terms of this assay, in close agreement with the 0.3 μM observed by ITC measurements (n = 5).

In the BROMO*scan*™ assay, the compound of formula (4) was found to bind specifically to the BET bromodomain family. It exhibited affinity values in the nM range for 3 members, namely BRD2-BD1 and both BRDs of BRD3, and low μM affinity to the other members ($K_d$ values between 1.5 and 3.5 μM).

So far, no molecules exhibiting binding selectivity within the BET family have been reported, probably due to the similarity of their binding pockets, both in terms of 3-dimensional structure and amino acid sequence. A recent classification of the BRD family pointed out that the CREBBP and EP300 BRDs had binding site amino acid signatures in common with the BET bromodomains. Indeed, the compound of formula (4) showed ~3.5 μM binding affinity to these two other BRDs. This result is remarkable, as only a few small molecules displaying significant binding to those proteins have been described so far. In the case of CREBBP, the compound ischemin ($K_d$ = 19 μM) was found to inhibit its association with p53, further blocking apoptosis in cardiomyocytes.

**Induction of apoptosis in leukaemia cells**

BRD4 is a validated therapeutic target in NUT midline carcinoma and in acute myeloid and MLL-fusion leukaemia. The proliferation inhibition potential of the virtual screening hits in HL-60 and HeLa cell lines for the compound of formula (4) was assessed. The compound of formula (4) exhibited a $GI_{50}$ value between 5 and 20 μM in HL-60 cells and no antiproliferative activity against HeLa at a high concentration of 50 μM.

The antiproliferative potential of the compound of formula (4) was also assessed by means of the NCI-60 DTP Human Tumour Cell Line Screen. This assay consisted of 56 cell lines representing 9 different cancer types. The results are summarized in Fig. 2.

Fig. 2 shows a NCI-60 DTP Human Tumour Cell Line Screen. Part A shows the growth inhibition (GI, %) observed after incubation of each cell line with the compound of formula (4) at a concentration of 10 μM. Part B shows a box plot representation of the proliferation inhibition (%) of the cell lines grouped by cancer type. The compound of formula (4) showed potent and selective activity against the leukaemia cell lines represented in the assay. Indeed, the most sensitive cells were HL-60(TB) and SR, from the leukaemia panel, whereas the most resistant were OVCAR-5 and COLO 205, from ovarian and colon cancer respectively. The Cell Line Screen results indicate that the compound of formula (4) selectively induces apoptosis in leukaemia cells.

Three descriptors to determine whether the compounds for use in accordance with the present invention have desirable drug-like properties were calculated. Quantitative estimate of drug-likeness (QED) is a recently developed measure of drug-likeness based on the concept of desirability of physicochemical descriptors present in drugs (Bickerton GR, Paolini GV, Besnard J, Muresan S, & Hopkins AL (2012) Quantifying the chemical beauty of drugs. Nature chemistry 4(2):90-98). Ligand efficiency indexes provide a measure of how efficiently a ligand binds to a biomolecule with respect to the magnitude of a physical property of the compound. Thus, we have computed the ligand efficiency in terms of number of heavy atoms (LE) and lipophilicity per unit of potency (LLE) (Hopkins AL GC, Alex A. (2004) Ligand efficiency: a useful metric for lead selection. Drug discovery today 9(10):430-431; Leeson PD & Springthorpe B (2007) The influence of drug-like concepts on decision-making in medicinal chemistry. Nature reviews. Drug discovery 6(11):881-890)

Table 1 summarizes the drug-likeness analysis for the compound of formula (4) and for the compound of formula (3) with R5 being methyl, and the reference compounds, JQ1 and I-BET. QED scores indicate that the four molecules are drug-beauty, with the compound of formula (4) and JQ1 having the lowest values in the range of 0.4. LE has proven useful in the estimation of the potency of a compound to disrupt a protein-protein complex (Wells JA & McClendon CL (2007) Reaching for high-hanging fruit in drug discovery at protein-protein interfaces. Nature 450(7172):1001-1009). It could be shown that potent inhibitors had LE values above 0.24. Interestingly, the compound of formula (4) has a LE of 0.31, similar to that of JQ1 and I-BET. The compound of formula (3) with $R_5$ being methyl, as a representative of the new 4-acyl pyrrole BET family inhibitors, displayed the highest ligand efficiency indexes among the studied compounds, with an LE value of 0.43 and LLE of 3.7. The analysis of the drug-likeness and physicochemical properties of the compound of formula (4) and the compound of formula (3) with R5 being methyl indicate that the 4-acyl pyrrole moiety is an interesting scaffold for the development of molecules binding to the BET bromodomains.

Table 1

Summary of drug-likeness and ligand efficiency index parameters computed for compound of formula (4), compound of formula (3), JQ1, and I-BET. $pK_d$, quantitative estimate of drug-likeness (QED), ligand efficiency (LE, calculated as 1,37($pK_d$/number of heavy atoms), $kcal \cdot mol^{-1} \cdot heavy\ atom^{-1}$), and ligand-lipophilicity efficiency (LLE, calculated as $pK_d$ - $clogP_{o/w}$) are shown

| | $pK_d$ | QED | LE | LLE |
|---|---|---|---|---|
| **Compound of formula (4)** | 6.5 | 0.41 | 0.31 | 2.8 |
| **Compound of formula (3)** | 4.7 | 0.73 | 0.43 | 3.7 |
| **JQ1** | 7.6 | 0.44 | 0.34 | 3.5 |
| **I-BET** | 7.3 | 0.65 | 0.33 | 3.9 |

## Claims

**1.** A compound of formula (1) or a pharmaceutically acceptable salt thereof

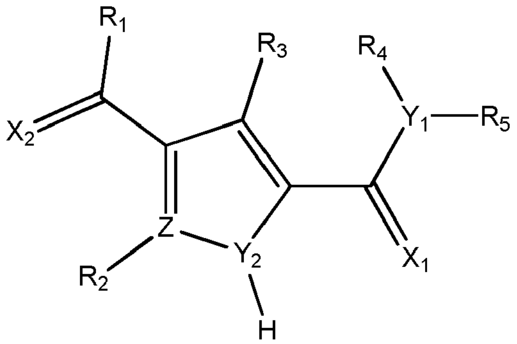

(1)

for use in the treatment of diseases or conditions for which a bromodomain inhibitor is indicated,
wherein
$Y_1$ and $Y_2$, which may be the same or different at each occurrence, are N or P, $X_1$ and $X_2$, which may be the same or different, are O or S,
Z is C, S, C-$CR_6R_7$ or C-S-
$R_1$ is $C_1$-$C_8$ alkyl, $NHR_8$, $PHR_8$, $NH_2$, $PH_2$, a substituted or unsubstituted $C_1$-$C_8$-hydroxyalkyl group, or a substituted or unsubstituted $C_1$-$C_8$-alkoxy group,
$R_2$ is hydrogen, a halogen atom, a cyano group or a thiocyanate or isothiocyanate group or a $C_1$ to $C_8$ alkyl group,
$R_3$ is hydrogen, $C_1$-$C_8$ alkyl or a substituted or unsubstituted $C_3$-$C_8$-carbocyclyl, a $C_3$-$C_8$ heterocarbocyclyl group with up to two heteroatoms selected from N, O or S, or a 5- or 6-membered aryl or heteroaryl group,
$R_4$ and $R_5$, which may be the same or different at each occurrence, are hydrogen, a $C_1$-$C_8$ alkyl group, a $C_1$-$C_8$ alkoxy group, a $C_1$-$C_8$ hydroxyalkyl group, a substituted or unsubstituted $C_3$-$C_{14}$ cycloalkyl or heterocycloalkyl group or an unsubstituted or substituted $C_3$-$C_{14}$ aryl or heteroaryl group, which cycloalkyl, heterocycloalkyl, aryl or heteroaryl group may be bound to $Y_1$ directly or through a spacer of a $C_1$-$C_8$ alkylene group
$R_6$ and $R_7$, which may be the same or different, are hydrogen or a $C_1$ to $C_8$ alkyl group and
$R_8$ is hydrogen or a $C_1$ to $C_8$ alkyl group.

**2.** A compound of formula (1) or a pharmaceutically acceptable salt thereof for use in accordance with claim 1 wherein $Y_1$ and/or $Y_2$ is N.

**3.** A compound of formula (1) or a pharmaceutically acceptable salt thereof for use in accordance with claim 1 or 2 wherein Z is C or C-S-, preferably C.

**4.** A compound of formula (1) or a pharmaceutically acceptable salt thereof for use in accordance with any of claims 1 to 3 wherein $R_4$ and/or $R_5$, which may be the same or different, are hydrogen or a substituted or unsubstituted

$C_3$-$C_{14}$ aryl group, which may be bound to $Y_1$ directly or through a spacer of a $C_1$-$C_8$ alkylene group.

**5.** A compound of formula (1) or a pharmaceutically acceptable salt thereof for use in accordance with claim 4 wherein $R_4$ and/or $R_5$, which may be the same or different, are a substituted phenyl group, which may be bound to $Y_1$ directly or through a spacer of a $C_1$-$C_8$ alkylene group.

**6.** A compound of formula (1) or a pharmaceutically acceptable salt thereof for use in accordance with claim 5 wherein $R_4$ and/or $R_5$ represent a substituent of formula (2)

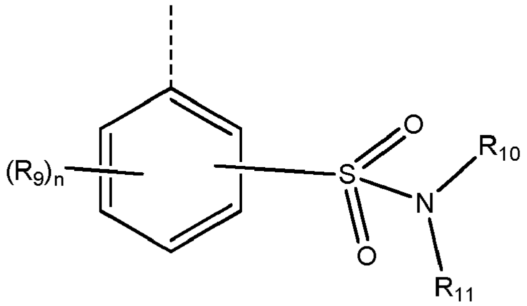

(2)

wherein $R_9$ is halogen, OH, SH, $SR_6$, cyano, thiocyanato, isothiocyanato, $NR_6R_7$, a $C_1$-$C_8$ alkyl group, a $C_2$-$C_8$ alkylene group, a $C_2$-$C_8$ alkynyl group or a $C_1$ to $C_8$ alkoxy group, wherein $R_6$ and $R_7$ are as previously defined and n is an integer of from 0 to 4, preferably of from 1 to 3, and
$R_{10}$ and $R_{11}$, which may be the same or different, are hydrogen, a $C_1$-$C_8$ alkyl group or a substituted or unsubstituted $C_3$-$C_{14}$ cycloalkyl group, heterocycloalkyl, aryl or heteroaryl group or wherein $R_{10}$ and $R_{11}$ may form together a cyclocarbyl, heterocyclocarbyl, aryl or heteroaryl ring, each with 3 to 14 carbon atoms.

**7.** A compound of formula (3) or a pharmaceutically acceptable salt thereof for use in accordance with claim 1

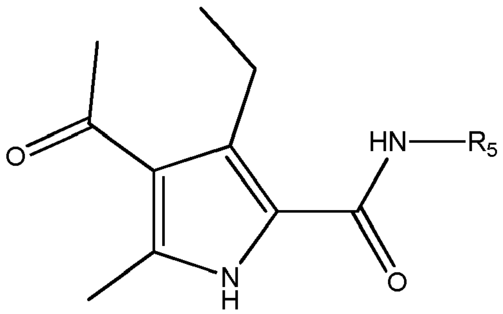

(3)

wherein R5 has the meaning as defined in claim 1.

**8.** A compound of formula (4) or a pharmaceutically acceptable salt thereof for use in accordance with claim 1

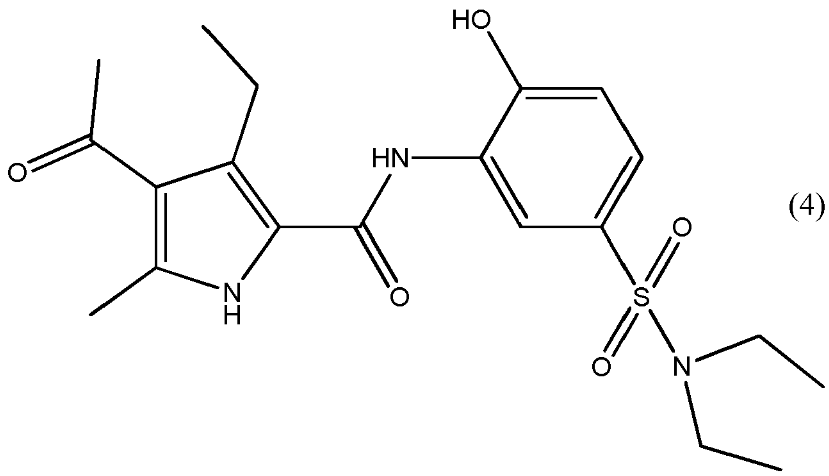

(4)

9. A compound of formula (1) or a pharmaceutically acceptable salt thereof for use according to any of claims 1 to 8 wherein the disease or condition is a chronic autoimmune or inflammatory condition, cancer or a viral disease.

10. A compound of formula (1) or a pharmaceutically acceptable salt thereof for use in accordance with claim for the induction of apoptosis in leukaemia cells.

11. A compound of formula (1) or a pharmaceutically acceptable salt thereof for use according to claim1 in accordance with any of claims 1 to 8 wherein the disease or condition is obesity.

12. A compound of formula (1) or a pharmaceutically acceptable salt thereof for use according to claim1 in accordance with any of claims 1 to 8 wherein the disease or condition is a kidney malfunction.

13. A compound of formula (1) or a pharmaceutically acceptable salt thereof for use according to claim1 in accordance with any of claims 1 to 8 as a male contraceptive.

14. A pharmaceutical composition comprising at least one compound of formula (1) or a pharmaceutically acceptable salt thereof and one or more pharmaceutically acceptable carriers, diluents or excipients.

15. A combination pharmaceutical product comprising at least one compound of formula (1) or a pharmaceutically acceptable salt thereof and one or more pharmaceutically acceptable carriers, diluents or excipients together with one or more other pharmaceutically active ingredients

Fig. 1

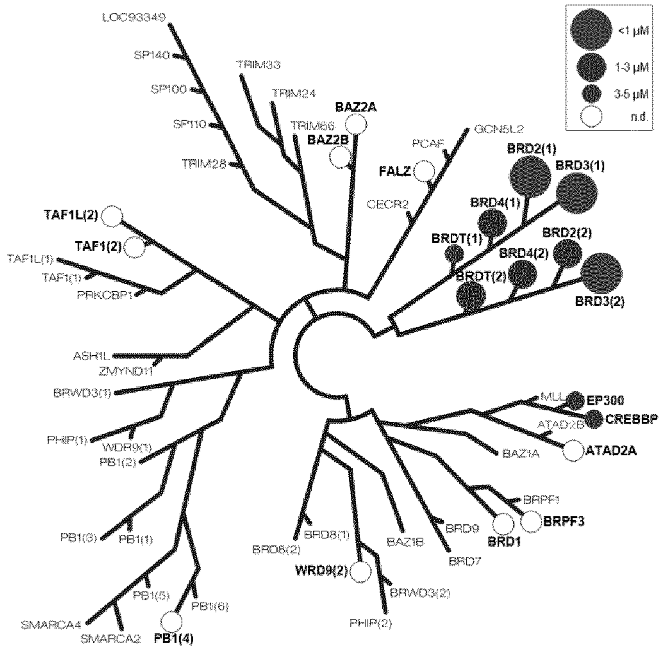


| | BRD2(1) | BRD2(2) | BRD3(1) | BRD3(2) | CREBBP |
|---|---|---|---|---|---|
| $K_d$ (µM) | 0.6 | 2.4 | 0.9 | 1.0 | 3.2 |

| | BRD4(1) | BRD4(2) | BRDT(1) | BRDT(2) | EP300 |
|---|---|---|---|---|---|
| $K_d$ (µM) | 2.7 | 2.3 | 3.4 | 1.4 | 4.1 |

Fig. 2

A

| | Cell line | GI (%) |
|---|---|---|
| Breast cancer | T-47D | 31.0 |
| | HS 578T | 18.0 |
| | BT-549 | 15.3 |
| | MCF7 | 15.1 |
| | MDA-MB-468 | 7.7 |
| | MDA-MB-231/ATCC | 2.4 |
| Colon cancer | KM12 | 24.3 |
| | HCT-116 | 10.9 |
| | SW-620 | 7.3 |
| | HCT-15 | 4.7 |
| | HT29 | 1.2 |
| | COLO 205 | -4.7 |
| Leukemia | HL-60(TB) | 65.0 |
| | SR | 64.2 |
| | K-562 | 42.0 |
| | MOLT-4 | 34.8 |
| | CCRF-CEM | 23.0 |
| | RPMI-8226 | 21.8 |
| Melanoma | UACC-62 | 23.5 |
| | M14 | 15.2 |
| | SK-MEL-5 | 12.9 |
| | UACC-257 | 10.3 |
| | MDA-MB-435 | 9.6 |
| | SK-MEL-2 | 9.1 |
| | SK-MEL-28 | 7.2 |
| | MALME-3M | 4.7 |
| Prostate cancer | PC-3 | 17.8 |
| | DU-145 | 2.6 |

| | Cell line | GI (%) |
|---|---|---|
| CNS cancer | SNB-75 | 29.4 |
| | SF-295 | 10.0 |
| | U251 | 6.6 |
| | SNB-19 | 1.3 |
| | SF-268 | 0.2 |
| Non-small cell lung cancer | NCI-H522 | 42.4 |
| | NCI-H322M | 25.4 |
| | HOP-92 | 15.8 |
| | NCI-H226 | 9.7 |
| | NCI-H23 | 8.9 |
| | HOP-62 | 5.7 |
| | A549/ATCC | 4.1 |
| | NCI-H460 | 3.2 |
| Ovarian cancer | IGROV1 | 27.3 |
| | OVCAR-4 | 14.8 |
| | OVCAR-3 | 11.2 |
| | OVCAR-8 | 6.7 |
| | SK-OV-3 | 6.4 |
| | NCI/ADR-RES | 3.2 |
| | OVCAR-5 | -6.7 |
| Renal cancer | UO-31 | 31.3 |
| | 786-0 | 15.6 |
| | A498 | 14.5 |
| | SN12C | 9.4 |
| | CAKI-1 | 6.1 |
| | ACHN | 3.6 |
| | TK-10 | -0.7 |
| | RXF 393 | -1.0 |

B

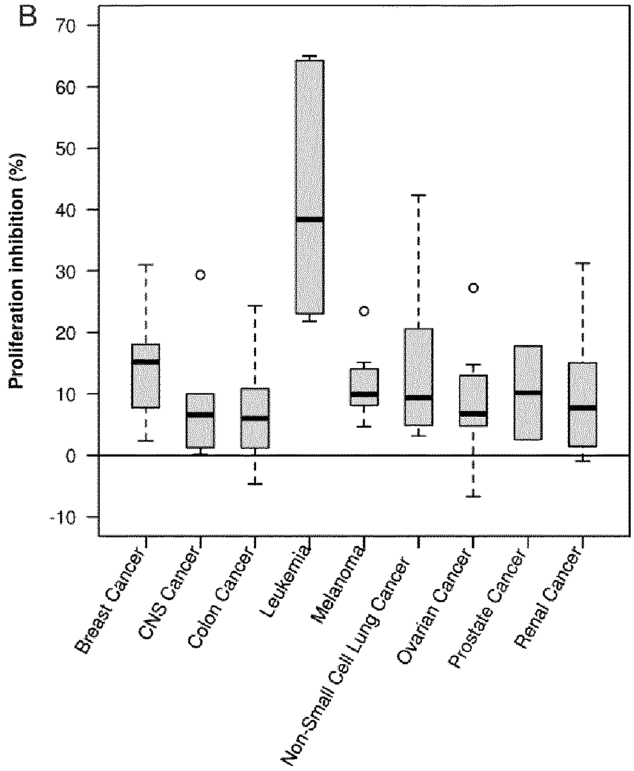

Europäisches Patentamt
European Patent Office
Office européen des brevets

# EUROPEAN SEARCH REPORT

Application Number
EP 13 16 4209

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2012/252139 A1 (BAMBOROUGH PAUL [GB] ET AL) 4 October 2012 (2012-10-04)<br>* the whole document *<br>----- | 1-15 | INV.<br>A61K31/40 |
| A,D | PRINJHA RK; WITHERINGTON J; LEE K: "Place your BETs: the therapeutic potential of bromodomains",<br>TRENDS IN PHARMACOLOGICAL SCIENCES,<br>vol. 33, no. 3, 2012, pages 146-153,<br>XP002711557,<br>* the whole document *<br>----- | 1-15 | |

| TECHNICAL FIELDS SEARCHED (IPC) |
|---|
| A61K |

The present search report has been drawn up for all claims

1

| Place of search | Date of completion of the search | Examiner |
|---|---|---|
| The Hague | 21 August 2013 | Langer, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 16 4209

This annex lists the patent family members relating to the patent documents cited in the above-mentioned European search report. The members are as contained in the European Patent Office EDP file on
The European Patent Office is in no way liable for these particulars which are merely given for the purpose of information.

21-08-2013

| Patent document cited in search report | Publication date | Patent family member(s) | Publication date |
|---|---|---|---|
| US 2012252139 A1 | 04-10-2012 | EP 2496945 A1 | 12-09-2012 |
| | | US 2012252139 A1 | 04-10-2012 |
| | | WO 2011054851 A1 | 12-05-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011054851 A
- WO 2012174487 A
- WO 2011054553 A
- JP 2008156311 A
- WO 2009084693 A
- WO 2012150234 A
- WO 97003957 A


### Non-patent literature cited in the description


- **FILIPPAKOPOULOS et al.** *Cell,* 2012, vol. 149 (1), 214-231
- **OWEN DJ et al.** The structural basis for the recognition of acetylated histone H4 by the bromodomain of histone acetyltransferase gcn5p. *The EMBO journal,* 2000, vol. 19 (22), 6141-6149
- **UMEHARA T et al.** Structural basis for acetylated histone H4 recognition by the human BRD2 bromodomain. *The Journal of biological chemistry,* 2010, vol. 285 (10), 7610-7618
- **MULLER S ; FILIPPAKOPOULOS P ; KNAPP S.** Bromodomains as therapeutic targets. *Expert reviews in molecular medicine,* 2011, vol. 13, e29
- **PRINJHA RK ; WITHERINGTON J ; LEE K.** Place your BETs: the therapeutic potential of bromodomains. *Trends in pharmacological sciences,* 2012, vol. 33 (3), 146-153
- **VIDLER LR ; BROWN N ; KNAPP S. ; HOELDER S.** Druggability analysis and structural classification of bromodomain acetyl-lysine binding sites. *Journal of medicinal chemistry,* 2012, vol. 55 (17), 7346-7359
- **BAMBOROUGH et al.** *J. Med. Chem.,* 2012, vol. 55, 587-596
- **HEWINGS et al.** *J. Med. Chem.,* 2011, vol. 54, 6761-6770
- **DAWSON et al.** *Nature,* 2011, vol. 478 (7370), 529-533
- **ZHAO et al.** Fragment based drug discovery of 2-thiazolidinones as inhibitors of the histone reader BRD4 bromodomain. *J. Med. Chem Just accepted,* 26 March 2013
- **NICODEME et al.** *Nature,* 2010, vol. 210, 1119-1123
- **BERGE et al.** 66. *J. Pharm.Sci.,* 1977, 1-19
- Burger's Medicinal Chemistry and drug discovery. Principles and Practice, vol. 1
- **FILIPPAKOPOULOS et al.** *Cell,* 2012, vol. 149 (1), 241-231
- **KABSCH W.** Xds. Acta crystallographica. *Section D, Biological crystallography,* 2010, vol. 66, 125-132
- **BRUKER.** SADABS, SAINT and XPREP. Bruker AXS Inc, 2008
- Acta crystallographica. *Section D, Biological crystallography,* vol. 50, 760-763
- **EVANS P.** Scaling and assessment of data quality. *Acta crystallographica. Section D, Biological crystallography,* 2006, vol. 62, 72-82
- **EVANS PR.** An introduction to data reduction: space-group determination, scaling and intensity statistics. *Acta crystallographica. Section D, Biological crystallography,* 2011, vol. 67, 282-292
- **MCCOY AJ et al.** Phaser crystallographic software. *Journal of applied crystallography,* 2007, vol. 40, 658-674
- **MURSHUDOV GN ; VAGIN AA ; DODSON EJ.** Refinement of macromolecular structures by the maximum-likelihood method. Acta crystallographica. *Section D, Biological crystallography,* 1997, vol. 53, 240-255
- **MURSHUDOV GN et al.** REFMAC5 for the refinement of macromolecular crystal structures. *Acta crystallographica. Section D, Biological crystallography,* 2011, vol. 67, 355-367
- **VAGUINE AA ; RICHELLE J ; WODAK SJ.** SFCHECK: a unified set of procedures for evaluating the quality of macromolecular structure-factor data and their agreement with the atomic model. *Acta crystallographica. Section D, Biological crystallography,* 1999, vol. 55, 191-205
- **WLODEK S ; SKILLMAN AG ; NICHOLLS A.** Automated ligand placement and refinement with a combined force field and shape potential. Acta crystallographica. *Section D, Biological crystallography,* 2006, vol. 62, 741-749
- **BICKERTON GR ; PAOLINI GV ; BESNARD J ; MURESAN S ; HOPKINS AL.** Quantifying the chemical beauty of drugs. *Nature chemistry,* 2012, vol. 4 (2), 90-98

- **HOPKINS AL GC ; ALEX A.** Ligand efficiency: a useful metric for lead selection. *Drug discovery today,* 2007, vol. 9 (10), 430-431
- **LEESON PD ; SPRINGTHORPE B.** The influence of drug-like concepts on decision-making in medicinal chemistry. *Nature reviews. Drug discovery,* 2007, vol. 6 (11), 881-890
- **WELLS JA ; MCCLENDON CL.** Reaching for high-hanging fruit in drug discovery at protein-protein interfaces. *Nature,* 2007, vol. 450 (7172), 1001-1009